# EUROPÄISCHE PATENTSCHRIFT

(11) **EP 1 304 130 B1**
(45) Veröffentlichungstag und Bekanntmachung des Hinweises auf die Patenterteilung: **23.06.2004**
(21) Anmeldenummer: 01124294.8
(22) Anmeldetag: 18.10.2001
(51) Int. Cl.: A61M 15/00, B05B 17/06, A61M 11/00, B05B 7/00, B06B 1/06, B06B 1/00, H03H 9/00

(54) **Inhalationstherapievorrichtung**
Inhalation therapy device
Dispositif de traitement par inhalation

(43) Veröffentlichungstag der Anmeldung: 23.04.2003
(73) Patentinhaber: PARI GmbH Spezialisten für effektive Inhalation, 82319 Starnberg (DE)
(72) Erfinder: Kunschir, Eduard, Dipl.-Ing., 80469 München (DE)
(74) Vertreter: HOFFMANN - EITLE

(56) Entgegenhaltungen:
- EP-A- 1 092 446
- DE-A- 19 962 280
- US-A- 4 300 131
- US-A- 4 790 479
- US-A- 5 487 378
- US-A- 6 152 130

## Beschreibung

Die Erfindung betrifft Inhalationstherapiegeräte mit einem Aerosolerzeuger, insbesondere mit einer schwingungsfähigen Membran zur Vernebelung einer Flüssigkeit oder eines Pulvers.

Bei Inhalationstherapiegeräten mit steuerbarem Aerosolerzeuger besteht der Bedarf, dem Patienten unterschiedliche Betriebszustände des Geräts zu signalisieren. Bislang werden dazu Anzeigeelemente, z.B. Leuchtdioden oder Signaltongeber eingesetzt, mit deren Hilfe dem Patienten z.B. der Anfang und das Ende einer Therapiesitzung oder andere Informationen angezeigt werden.

Der nächstliegende Stand der Technik ist im Dokument US-A-5 487 378 offenbart.

Durch die Erfindung wird ein Weg aufgezeigt, wie bei einer Inhalationstherapievorrichtung ohne zusätzlichen Signaltongeber akustische Signale abgegeben werden können, z.B. um dem Patienten bestimmte Betriebszustände der Vorrichtung anzuzeigen.

Erfindungsgemäß wird dies erreicht durch eine Inhalationstherapievorrichtung mit einer schwingungsfähigen Membran für die Vernebelung einer Flüssigkeit, mit einer Schwingungserzeugungseinrichtung, die zumindest eine Anschlusseinrichtung für die Zuführung eines Schwingungsansteuerungssignals aufweist und durch die die Membran in Schwingungen versetzt wird, wenn das Schwingungsansteuerungssignal zugeführt wird, so dass eine auf einer Seite der Membran anstehende Flüssigkeit durch die Membran hindurch vernebelt wird und auf der anderen Seite der Membran als Aerosol vorliegt, und mit einer Steuereinrichtung, von der ein Schwingungsansteuerungssignal der zumindest einen Anschlusseinrichtung der Schwingungserzeugungseinrichtung zuführbar ist, so dass die Schwingungserzeugungseinrichtung die Membran in Schwingungen versetzt, wobei die Steuereinrichtung derart ausgelegt ist, dass der Schwingungserzeugungseinrichtung ein weiteres Ansteuerungssignal von der Steuereinrichtung gleichzeitig mit dem ersten zuführbar ist, das die Membran im hörbaren Frequenzbereich zur Abgabe eines hörbaren Signals für einen Benutzer in Schwingungen versetzt.

Die Erfindung beruht auf der durchaus überraschenden Erkenntnis, dass bei Membranverneblern die für die Schwingungserzeugung eingesetzte elektro-mechanische Wandlereinheit, in der Regel ein piezoelektrisches Bauteil, auch zu Schwingungen im hörbaren Frequenzbereich angeregt werden kann und die Verneblermembran dann als elektro-akustischer Wandler (Signalgeber) arbeitet, ohne dass die Vernebelung beeinträchtigt wird. Überraschend ist auch, dass ausreichend hohe Signalpegel erreichbar sind, und zwar auch während des Vernebelungsbetriebs.

Im folgenden wird die Erfindung anhand eines Ausführungsbeispiels unter Bezugnahme auf die Figur genauer erläutert, die eine schematische Darstellung einer erfindungsgemäßen Inhalationstherapievorrichtung zeigt.

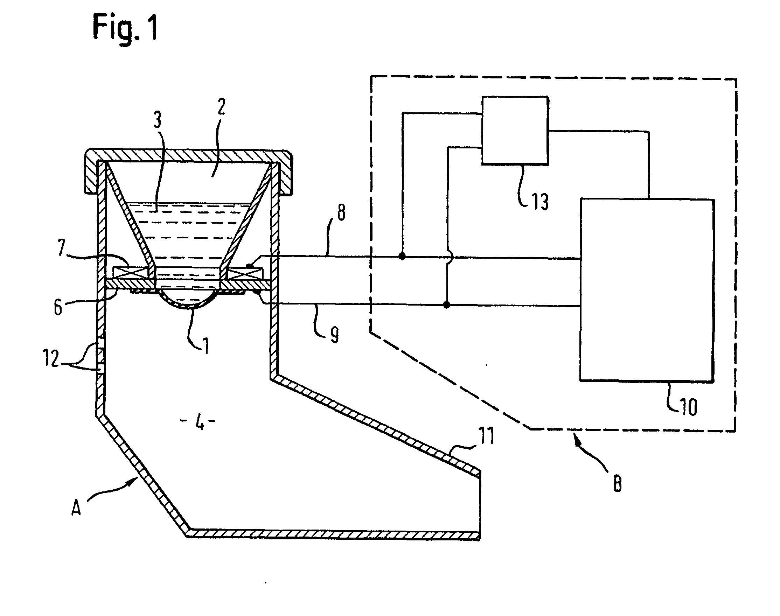

In Fig. 1 ist ein Ausführungsbeispiel einer erfindungsgemäßen Inhalationstherapievorrichtung dargestellt, bei der in einer Verneblereinheit A mit Hilfe einer Membran 1 eine in einem Flüssigkeitsreservoir 2 bevorratete Flüssigkeit 3 in einen Vernebelungshohlraum 4 hinein vernebelt wird. Die Vernebelung findet dann statt, wenn die Membran 1 in Schwingungen versetzt wird. Dazu ist die Membran 1 an einer Supporteinheit 6 befestigt, die die Membran 1 trägt und an der auch eine elektro-mechanische Wandlereineinheit 7, beispielsweise ein Piezoelement, befestigt ist. Die Membran 1, die Supporteinheit 6 und die elektro-mechanische Wandlereinheit 7 sind bei dem hier beschriebenen Ausführungsbeispiel rotationssymmetrisch ausgebildet und bilden zusammen ein schwingungsfähiges Gebilde. Über Anschlussleitungen 8 und 9 kann an die elektro-mechanische Wandlereinheit 7 ein Ansteuerungssignal von einer Steuereinrichtung 10 zugeführt werden, die bei dem hier beschriebenen Ausführungsbeispiel in einer separaten Steuerungseinheit B untergebracht ist. Wenn das Ansteuerungssignal zugeführt wird, wird das schwingfähige Gebilde 1, 6, 7 in Schwingungen versetzt und die Flüssigkeit 3 durch die Membran 1 hindurch vernebelt.

Ein Patient kann das im Vernebelungshohlraum 4 bereitgestellte Aerosol am Mundstück 11 des Verneblers einatmen. Für die Zuführung einer ausreichenden Atemluftmenge sind im Gehäuse des Verneblers ein oder mehrere Atemöffnungen 12 vorgesehen, durch die Umgebungsluft in den Hohlraum 4 während des Einatmens eintreten und aus denen die Atemluft des Patienten aus dem Hohlraum 4 während des Ausatmens austreten kann.

Um dem Patienten anzuzeigen, dass das Inhalationstherapiegerät sich in einem definierten Betriebszustand befindet, wird erfindungsgemäß ein weiteres Ansteuerungssignal dem schwingfähigen Gebilde 1, 6, 7 zugeführt. Das weitere Ansteuerungssignal versetzt die Membran 1 in Schwingungen bei einer hörbaren Frequenz, so dass die Membran 1 einen für den Patienten hörbares Tonsignal abgibt. Die Abgabe des Signaltons kann während des Vernebelungsbetriebs der Membran 1 erfolgen, ohne dass sich die beiden Schwingungen stören. Dies ist überraschend, da für die Abgabe eines hörbaren Tons nicht nur eine Frequenz aus dem hörbaren Bereich gewählt werden muss, sondern weil auch eine ausreichende Energie in das schwingfähige Gebilde 1, 6, 7 zugeführt werden muss, damit in wahrnehmbaren Umfang Schallenergie abgestrahlt werden kann. Dennoch wird die Vernebelung der Flüssigkeit und die Qualität des erzeugten Aerosols nicht negativ beeinträchtigt, zumal sich die Signaltonabgabe in der Regel auf vergleichsweise kurze Zeiträume beschränkt.

So kann z.B. ein kurzer Signalton von 0,5 bis 2 s Dauer abgegeben werden, wenn nach dem Einschalten die optimale Betriebsfrequenz des schwingfähigen Gebildes 1, 6, 7 durch eine Suchlaufschaltung gefunden wurde und sich die Membran in einem eingeschwungenen Zustand befindet. Dem Patienten kann dann durch den Signalton angezeigt werden, dass die Inhalationstherapievorrichtung betriebsbereit ist und die Therapiesitzung beginnen kann. Ebenso kann nach einem vorgegebenen Zeitraum oder nach dem Vernebeln einer vorbestimmten Flüssigkeitsmenge das Ende der Therapiesitzung durch einen vorzugsweise anders klingenden Signalton signalisiert werden. Die Signaltöne sind nicht auf reine Töne beschränkt, vielmehr können auch Tonfolgen oder aufgezeichnete oder synthetische Sprachsignale zum Einsatz kommen.

Für die Erzeugung des weiteren Ansteuerungssignals ist vorzugsweise eine Generatorschaltung 13 vorgesehen, von der das weitere Ansteuerungssignal dem schwingfähigen Gebilde 1, 6, 7 zugeführt wird. Dazu werden beim dem hier beschriebenen Ausführungsbeispiel die beiden vorhandenen Anschlussleitungen 8 und 9 verwendet, über die auch das Schwingungsansteuerungssignal dem schwingfähigen Gebilde 1, 6, 7 zugeführt wird. Die Generatoreinheit 13 ist vorteilhafterweise in die Steuereinrichtung 10 integriert.

Das oben genauer beschriebene Ausführungsbeispiel zeigt, wie die erfindungsgemäße Erzeugung eines akustischen Signals für den Patienten bei einem Inhalationstherapiegerät mit Membranvernebler erfolgt. Die Beschreibung des Ausführungsbeispiels macht aber auch deutlich, dass die Erfindung bei allen Inhalationstherapiegeräten angewendet werden kann, bei denen eine Aerosolerzeugungseinrichtung mit einem Ansteuerungssignal beaufschlagt wird, um ein Aerosol zu erzeugen, und bei denen ein schwingfähiges Gebilde eingesetzt wird, das neben den für die Vernebelung erforderlichen Schwingungen zu Schwingungen im hörbaren Bereich angeregt werden kann.

In besonderem Maße eignen sich zur Anwendung der Erfindung Inhalationstherapiegeräte, bei denen eine elektro-mechanische Wandlereinheit vorzugsweise in Form eines piezoelektrischen Elements vorgesehen ist.

## Patentansprüche

1. Inhalationstherapievorrichtung
a. mit einer schwingungsfähigen Membran (1) für die Vernebelung einer Flüssigkeit (3),
b.mit einer Schwingungserzeugungseinrichtung (6,7), die zumindest eine Anschlusseinrichtung (8,9) für die Zuführung eines Schwingungsansteuerungssignals aufweist und durch die die Membran (1) in Schwingungen versetzt wird, wenn das Schwingungsansteuerungssignal zugeführt wird, so dass eine auf einer Seite der Membran anstehende Flüssigkeit durch die Membran hindurch vernebelt wird und auf der anderen Seite der Membran als Aerosol vorliegt, und
c. mit einer Steuereinrichtung (10), von der ein Schwingungsansteuerungssignal der zumindest einen Anschlusseinrichtung (8,9) der Schwingungserzeugungseinrichtung (6,7) zuführbar ist, so dass die Schwingungserzeugungseinrichtung (6,7) die Membran (1) in Schwingungen versetzt:,
**dadurch gekennzeichnet, dass**
die Steuereinrichtung (10) derart ausgelegt ist, dass der Schwingungserzeugungseinrichtung (6,7) ein weiteres Ansteuerungssignal von der Steuereinrichtung (10) gleichzeitig mit dem ersten zuführbar ist, das die Membran (1) im hörbaren Frequenzbereich zur Abgabe eines hörbaren Signals für einen Benutzer in Schwingungen versetzt.

2. Inhalationstherapievorrichtung nach Anspruch 1, **dadurch gekennzeichnet, dass** das weitere Ansteuerungssignal der Schwingungserzeugungseinrichtung (6,7) über dieselbe Anschlusseinrichtung (8,9) wie das Schwingungsansteuerungssignal zugeführt wird.

3. Inhalationstherapievorrichtung nach Anspruch 1, **dadurch gekennzeichnet, dass** die Schwingungserzeugungseinrichtung (6,7) eine elektro-mechanische Wandlereinheit (7), insbesondere ein piezo-elektrisches Element umfasst.

4. Inhalationstherapievorrichtung nach Anspruch 2, **dadurch gekennzeichnet, dass** die Schwingungserzeugungseinrichtung (6, 7) eine Supporteinheit (6) umfasst, an der die elektro-mechanische Wandlereinheit (6) und die Membran (1) befestigt sind.

5. Inhalationstherapievorrichtung nach einem der vorangegangenen Ansprüche, **dadurch gekennzeichnet, dass** eine Generatoreinheit (13) vorgesehen ist, die das weitere Ansteuerungssignal erzeugt, das über die zumindest eine Anschlusseinrichtung (8,9) der Schwingungserzeugungseinrichtung (6,7) zugeführt wird.

6. Inhalationstherapievorrichtung nach Anspruch 4, **dadurch gekennzeichnet, dass** die Generatoreinheit (13) in die Steuereinrichtung (10) integriert ist.

7. Inhalationstherapievorrichtung nach einem der vorangegangenen Ansprüche, **dadurch gekennzeichnet, dass** in der Steuereinrichtung (10) eine Energieversorgungseinrichtung für die Inhalationsvorrichtung integriert ist.

## Claims

1. Inhalation therapy device
a. having a membrane (1) which can be vibrated for atomisation of a liquid (3),
b. having a vibration-producing device (6, 7), which has at least one connection device (8, 9) for the supply of a vibration-control signal and by means of which the membrane (1) is vibrated if the vibration-control signal is supplied, so that a liquid waiting on one side of the membrane is atomised through the membrane and is present as an aerosol on the other side of the membrane, and
c. having a control device (10), from which a vibration-control signal of the at least one connection device (8, 9) of the vibration-producing device (6, 7) can be supplied, so that the vibration-producing device (6, 7) vibrates the membrane (1),
**characterised in that** the control device (10) is designed such that a further control signal from the control device (10), which vibrates the membrane (1) in the audible frequency range to emit an audible signal for a user, can be supplied at the same time as the first to the vibration-producing device (6, 7).

2. Inhalation therapy device according to claim 1, **characterised in that** the further control signal is supplied to the vibration-producing device (6, 7) via the same connection device (8, 9) as the vibration-control signal.

3. Inhalation therapy device according to claim 1, **characterised in that** the vibration-producing device (6, 7) comprises an electro-mechanical transducer unit (7), in particular a piezo-electric element.

4. Inhalation therapy device according to claim 2, **characterised in that** the vibration-producing device (6, 7) comprises a support unit (6), to which the electro-mechanical transducer unit (6) and the membrane (1) are attached.

5. Inhalation therapy device according to one of the preceding claims, **characterised in that** a generator unit (13) is provided, which produces the further control signal which is supplied to the vibration-producing device (6, 7) via the at least one connection device (8, 9).

6. Inhalation therapy device according to claim 4, **characterised in that** the generator unit (13) is integrated into the control device (10).

7. Inhalation therapy device according to one of the preceding claims, **characterised in that** an energy-supply device for the inhalation device is integrated in the control device (10).

## Revendications

1. Dispositif de traitement par inhalation,
a. avec une membrane (1), susceptible de vibrer pour produire la nébulisation d'un liquide (3),
b. avec un dispositif générateur de vibration (6, 7), présentant au moins un dispositif de raccordement (8, 9), pour l'amenée d'un signal de commande de vibration, et au moyen duquel la membrane (1) est amenée à vibrer lorsque le signal de commande de vibration est amené, de sorte qu'un liquide, appliqué sur une face de la membrane, soit nébulisé en traversant la membrane et se présente sur l'autre face de la membrane sous forme d'aérosol, et
c. avec un dispositif de commande (10), dont un signal de commande de vibration est susceptible d'être amené au au moins un dispositif de raccordement (8, 9) du dispositif de génération de vibration (6, 7), de manière que le dispositif de génération de vibration (6, 7) fasse vibrer la membrane (1),
**caractérisé en ce que**
le dispositif de commande (10) est conçu de manière que, au dispositif de génération de vibration (6, 7), puisse être amené un autre signal de commande, depuis le dispositif de commande (10), simultanément au premier, signal de commande qui fasse vibrer la membrane (1) dans la plage des fréquences audibles, dans le but de délivrer un signal audible à un utilisateur.

2. Dispositif de traitement par inhalation selon la revendication 1, **caractérisé en ce que** l'autre signal de commande est amené au dispositif de génération de vibration (6, 7) par l'intermédiaire du même dispositif de raccordement (8, 9) que celui servant au signal de commande de vibration.

3. Dispositif de traitement par inhalation selon la revendication 1, **caractérisé en ce que** le dispositif de génération de vibration (6, 7) comprend une unité de conversion électro-mécanique (7), en particulier un élément piézoélectrique.

4. Dispositif de traitement par inhalation selon la revendication 2, **caractérisé en ce que** le dispositif de génération de vibration (6, 7) comprend une unité support (6), à laquelle sont fixées l'unité de conversion électro-mécanique (6) et la membrane (1).

5. Dispositif de traitement par inhalation selon l'une des revendications précédentes, **caractérisé en ce qu'**est prévue une unité génératrice (13), générant un autre signal de commande, amené au dispositif de génération de vibration (6, 7) par l'intermédiaire du au moins un dispositif de raccordement (8, 9).

6. Dispositif de traitement par inhalation selon la revendication 4, **caractérisé en ce que** l'unité génératrice (13) est intégrée dans le dispositif de commande (10).

7. Dispositif de traitement par inhalation selon l'une des revendications précédentes, **caractérisé en ce qu'**un dispositif d'alimentation en énergie, pour le dispositif d'inhalation, est intégré dans le dispositif de commande (10).
